(19) Europäisches Patentamt
European Patent Office
Office européen des brevets

(11) Veröffentlichungsnummer: **0 022 206**
**B1**

(12) **EUROPÄISCHE PATENTSCHRIFT**

(45) Veröffentlichungstag der Patentschrift: **15.02.84**

(21) Anmeldenummer: **80103501.5**

(22) Anmeldetag: **23.06.80**

(51) Int. Cl.³: **C 07 D 307/54,** C 07 D 261/08, C 07 D 213/55, C 07 D 277/30, C 07 D 209/20, C 07 D 307/56, C 12 P 17/02, C 12 P 17/10, C 12 P 17/14

# (54) Optisch reine heterocyclische Aminosäuren, Verfahren zu ihrer Herstellung und ihre Verwendung.

(30) Priorität: **07.07.79 DE 2927534**

(43) Veröffentlichungstag der Anmeldung: **14.01.81 Patentblatt 81/2**

(45) Bekanntmachung des Hinweises auf die Patenterteilung: **15.02.84 Patentblatt 84/7**

(84) Benannte Vertragsstaaten: **AT BE CH DE FR GB IT LI**

(56) Entgegenhaltungen:
**DE - A - 2 419 838**
**DE - A - 2 707 812**
**US - A - 3 536 726**

**CHEMICAL ABSTRACTS, Band 58, Nr.11, 27.Mai 1963, Spalte 11464e, Columbus, Ohio, USA S. NISHIMURA et al. "Synthesis of norleucine and isoleucine from thiophene".**
**JOURNAL OF ORGANIC CHEMISTRY, Band 44, Nr. 3, 2.Februar 1979 Washington, US M.T. EDGAR et al. "Synthesis of L-(5-chloro-2-pyridyl) glycine", Seiten 396-400**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Preiss, Michael, Dr., Egenstrasse 21, D-5600 Wuppertal 1 (DE)**
Erfinder: **Schutt, Hermann, Dr., Roeberstrasse 8, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 022 206 B1**

# Optisch reine heterocyclische Aminosäuren, Verfahren zu ihrer Herstellung und ihre Verwendung

Die vorliegende Erfindung betrifft optisch reine heterocyclische Aminosäuren, ein enzymatisches Verfahren zu ihrer Herstellung durch stereoselektive Spaltung geeigneter heterocyclischer D, L-Aminosäure-Derivate und anschliessende Umwandlung in die D- bzw. L-Aminosäuren sowie ihre Verwendung als Zwischenprodukte für die Herstellung von Arzneimitteln.

Die nach dem erfindungsgemässen Verfahren hergestellten Verbindungen entsprechen der Formel

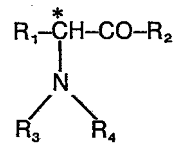

(I)

worin $R_1$ einen gesättigten oder ungesättigten gegebenenfalls substituierten heterocyclischen Rest, der 1 bis 4 gleiche oder verschiedene Heteroatome aus der Reihe Sauerstoff, Schwefel und/oder Stickstoff enthält, und gegebenenfalls benzokondensiert ist,

$R^2$ Hydroxy, $C_1$–$C_4$-Alkoxy oder $N(R_5)_2$,

$R_3$ und $R_4$ Wasserstoff, Acyl oder durch $C_1$–$C_4$-Alkoxycarbonyl substituiertes $C_2$–$C_4$-Alkenyl und

$R_5$ Wasserstoff oder $C_1$–$C_4$-Alkyl bedeuten und das mit * gekennzeichnete C-Atom entweder in der D- oder L-Form vorliegt.

Geeignete heterocyclische Reste sind beispielsweise Pyrazolyl, Imidazolyl, Oxazolyl, Oxdiazolyl, Thiazolinyl, Tetrazolyl, Sydnonyl, Pyridyl, Pyrazyl, Pyrimidinyl Pyridazyl, Chinolyl, Isochinolyl, Chinazolyl, Indolyl und Indazolyl sowie besonders bevorzug Furyl, Thienyl, Pyrrolyl Thiazolyl, Isothiazolyl, Isoxylyl und Thiadiazolyl. Die heterocyclischen Reste können einen oder mehrere vorzugsweise 1 bis 2 verschiedene oder insbesondere gleiche Substituenten tragen, wobei als Substituenten beispielsweise Halogen, wie Fluor, Chlor und Brom, Alkyl mit 1 bis 6 vorzugsweise 1 bis 4 C-Atomen, Amino Nitro, Cyano Sulfo, $C_1$–$C_4$-Alkylsulfonyl, Hydroxy oder Oxo in Frage kommen. Geeignetes Acyl $R_3$ und $R_4$ ist insbesondere gegebenenfalls substituiertes $C_1$–$C_6$-Alkylcarbonyl oder $C_1$–$C_6$-Alkoxycarbonyl, wobei als Substituenten gegebenenfalls durch Methyl, Methoxy, Chlor, Brom, Nitro oder Cyano substituiertes Phenyl, Carboxy oder Amino in Frage kommen.

Vorzugsweise steht $R_3$ für Wasserstoff und $R_4$ für Wasserstoff, Formyl, Acetyl, tert.-Butoxycarbonyl, Benzyloxycarbonyl oder 1-Methyl-2-$C_1$–$C_4$ Alkoxycarbonylvinyl. Besonders bevorzugt werden erfindungsgemäss hergestellt.

D-α-(5-Methyl-isoxazol-3-yl)-glycin-hydrochlorid,
D-α-Pyrid-3-yl-glycin-dihydrochloird,
D-α-Thiazol-2-yl-glycin-hydrochlorid
und D-α-5-Chlorfuryl-glycin-hydrochlorid.

Das erfindungsgemässe Verfahren zur Herstellung der Verbindungen der Formel I ist dadurch gekennzeichnet, dass man auf die racemischen Verbindungen der Formel I, worin jedoch $R_2$ nicht Hydroxy und $R_4$ nicht Wasserstoff bedeuten, trägergebundene, proteolytische Enzyme aus der Gruppe Subtilisin, α-Chymotrypsin, Papain, Ficin, Bromelain oder aus Bacillus subtilis oder Bacillus licheniformis isolierte proteolytische Enzyme in einem zweiphasigen Medium, bestehend aus Wasser und einem mit Wasser nicht mischbaren Lösungsmittel, einwirken lässt, die unveränderten D-Verbindungen der Formel I, worin $R_2$ nicht Hydroxy und $R_4$ nicht Wasserstoff ist, von den L-Verbindungen der Formel I, worin $R_2$ Hydroxy ist und $R_4$ nicht Wasserstoff ist, in üblicher Weise abtrennt und die voneinander getrennten D- und L-Verbindungen gegebenenfalls in üblicher Weise in Verbindungen der Formel I, worin $R_2$ Hydroxy und $R_3$ und $R_4$ Wasserstoff bedeuten, überführt.

Das erfindungsgemässe Verfahren beruht also darauf, in den L-Verbindungen die Ester- bzw. Amid-Gruppe $CO$–$R_2$ in die Carboxygruppe zu überführen, während die D-Verbindung dieser Hydrolyse nicht unterliegt. Soll die D-Form optisch rein erhalten werden, so ist auf die vollständige Hydrolyse des L-Derivates zu achten; soll hingegen die L-Form optisch rein gewonnen werden, so sollte die Hydrolyse der L-Ester und Amide nicht vollständig durchgeführt werden.

Die optisch reinen Verbindungen der Formel I dienen zur Herstellung von optisch reinen Penicillinen und Cephalosporinen, die bisher nur als Epimeren-Gemische zur Verfügung standen und beispielsweise aus den deutschen Offenlegungsschriften 2 732 283 und 2 810 083 bekannt sind. Weiterhin können die erfindungsgemässen Verbindungen auch zur Herstellung bisher nicht bekannter Penicilline und Cephalosporine verwendet werden.

In der Literatur sind bisher einige Beispiele für eine stereospezifische enzymatische Hydrolyse von N-Acyl-L-Aminosäureestern durch Subtilisin oder Chymotrypsin bekanntgeworden. Die beschriebenen Fälle betreffen Spaltungen von Derivaten natürlicher, vornehmlich in Proteinen vorkommender Aminosäuren. Für diese Aminosäuren war eine Spaltung anhand der bekannten Spezifität der verwendeten proteolytischen Enzyme mehr oder weniger vorhersehbar (A. Berger, M. Smolarski, M. Kun und H.D. Bosshard, J. Org. Chem., 3838 (1973) 457–460, DE-OS 2 804 892, US-PS 3 963 573, A.O. Barel und A.N. Glazer, J. Biol. Chem., 243 (1968), 1344–1348, K. Morihara und H. Tsuzuki, Arch. Biochem. Biophys., 129 (1969), 620–634, T.N. Pattabiraman und W.B. Lawson, Biochem. J., 126 (1972), 645–657).

Eine Trennung der optischen Antipoden der heterocyclischen Aminosäuren der Formel I war bis jetzt in der Literatur noch nicht beschrieben worden. Nach Chemical Reviews 46 (1950), 69–153, insbesondere 119–122 waren die erfindungsgemäss erhaltenen Ergebnisse auch nicht zu erwarten, weil danach proteolytische Enzyme, wie Subtilisin und Chymotrypsin, als ungeeignet angesehen werden mussten. Nach dieser Literaturstelle sind dann N-Acyl-Aminosäureester als Substrate von Chymotrypsin (und Subtilisin) spaltbar, wenn der aromatische Ring mindestens durch eine Methylengruppe vom α-Kohlenstoffatom getrennt ist (S. 119 Mitte). Derivate, in denen das Ringsystem direkt an das α-Kohlenstoffatom gebunden ist, dürften demnach nicht enzymatisch spaltbar sein. Benzoyl-DL-Phenylglycin ist sogar ein sehr starkter Inhibitor des Enzymes (S. 122, Mitte). Die verwendeten Enzyme sind proteolytische Enzyme aus der Gruppe Subtilisin (EC 3.4.4.16.), α-Chymotrypsin (EC 3.4.4.5.), Papain (EC 3.4.4.10.), Ficin oder Bromelain, wobei die ersten beiden einen Serinrest im aktiven Zentrum der Aminosäurekette haben, während letztere in der Aminosäurekette Cystein als aktives Zentrum aufweisen. Subtilisin ist bevorzugt.

Besonders geeignet sind aus Bacillus subtilis und Bacillus licheniformis isolierte proteolytische Enzyme, die den Waschmitteln zur Entfernung von Proteinresten zugesetzt werden. Diese technischen Enzyme sind vornehmlich unter den Firmennamen Maxatase(R) (Hersteller: Gist-Brocades N.V. Delft/Niederlande), Optimase(R), Hersteller: Miles-Kali Chemie, Hannover) und Alcalase(R), Hersteller: Novo Industrie AS, Kopenhagen/Dänemark, bekannt.

Die Eigenschaften der proteolytischen Enzyme, insbesondere ihre biochemischen Wirkungen, sind in folgenden Literaturstellen beschrieben: G.E. Perlmann und L. Lorand, Methods in Enzymology, 19 (1970) 199–215 . P. Desnuelle, The Enzymes, 4 (1960) 93 und G.E. Perlmann und L. Lorand, Methods in Enzymology, 19 (1970) 226–244.

Die proteolytischen Enzyme können durch eine kovalente Bindung über einen zur Katalyse nicht essentiellen Lysinrest an den poylmeren Träger gekuppelt werden. Die durch kovalente Bindung des Enzyms an den Träger entstehenden trägergebundenen Enzyme verlieren nur sehr langsam nach vielfacher Wiederverwendung ihre Aktivität. Eine weitere Möglichkeit ist, die Adsorption des Enzyms an die Poren eines geladenen Trägers sowie die anschliessende Quervernetzung mit Glutardialdehyd.

Als Enzymträger kommen polymere, poröse Träger wie Cellulosen, z.B. DEAE- oder CM-Cellulose, modifizierte Polyacrylamidgele mit Amino- oder Hydroxylgruppen oder organische Copolymerisate aus Acrylamid, Methacrylaten oder Methacrylamid und Maleinsäureanhydrid nach den DE-OS 2 215 539 und 2 215 687 in Frage.

Das Enzym wird zur Kupplung mit dem polymeren Träger unter optimalen Bedingungen für die Stabilität des Enzyms zur Reaktion gebracht. Die Effektivität der Kupplung kann durch Messung der enzymatischen Aktivität am Polymer und im Waschwasser bestimmt werden. Das polymere Enzym kann bei Verwendung im Batch-Verfahren leicht durch Sedimentation oder Filtration von der Reaktionslösung abgetrennt und mehrfach eingesetzt werden. Der Enzymträger kann auch in Säulen gefüllt und in Gegenwart eines Puffersystems von Substratlösung durchströmt werden.

Voraussetzung zur Eignung eines Enzymträgers für das erfindungsgemässe Verfahren ist ein vorheriger Test zur Adsorption der Ausgangs- und Endprodukte. Geeignete Träger dürfen die Ausgangs- und Endprodukte nur in ganz geringem Umfang oder gar nicht adsorbieren.

Als besonders gute Träger für die proteolytischen Enzyme haben sich die verschiedenen Cellulosen, derivatisierten Cellulosen Amino- und Hydroxylgruppen-haltigen Polyacrylamidgele sowie die durch Amino- oder Hydroxylgruppen modifizierten Anhydridharze erwiesen. Generell können als Träger alle Amino- und Hydroxylgruppen tragenden polymeren Träger eingesetzt werden, die die Ausgangs- und Endprodukte des erfindungsgemässen Verfahrens nicht adsorbieren. Der polymere Träger wird nach an sich bekannten Methoden mit Cyanurchlorid (GB-PS 1 302 706, N.L. Smith und H.M. Lohnhoff, Arch. Biochem., 61 (1974) 392–415, T.H. Finlay et al., Arch. Biochem., 87 (1978) 77–90) oder verschiedenen Halogenpyrimidinen nach DE-OS 2 619 521 oder DE-OS 2 619 451 aktiviert.

Die in dem erfindungsgemässen Verfahren eingesetzten N-Acylester und -Amide der heterocyclischen Aminosäuren werden durch Acylieren der entsprechenden Aminosäureester- bzw. -amidhydrochloride mit stöchiometrischen Mengen Säureanhydrid wie Acetanhydrid und anschliessende Abtrennung des N-Acylderivates aus der wässrigen Phase mit organischen Lösungsmitteln wie Chloroform oder Methylenchlorid gewonnen.

Die enzymatische Spaltung erfolgt vorzugsweise bei einer Temperatur von 20–40 °C in einem pH-Bereich von 6 bis 8, wobei der pH-Wert vorzugsweise durch Zugabe einer starken Base bei 7,0 konstant gehalten wird. Das Substrat wird als ca. 10–20%ige organische Lösung zu dem in Wasser suspendierten Enzym-Täger zugesetzt, wobei der Lösungsmittelanteil, bezogen auf das Gesamtvolumen, maximal 75–80 Vol.-% betragen kann. Das Reaktionsmedium wird während der enzymatischen Umsetzung intensiv gerührt. Der Verlauf und der Endpunkt der enzymatischen Reaktion kann durch die Neutralisation der entstehenden $H^+$-Ionen bestimmt werden. Die Neutralisation kann sowohl durch anorganische als auch durch organische Basen erfolgen.

Als organische Lösungsmittel für das erfindungsgemässe Verfahren kommen mit Wasser nicht mischbare Lösungsmittel wie Methylenchlorid, Chloroform, Toluol, Benzol, Essigsäureäthylester, Petroläther, Methylisobutylketon oder Isobutanol in Frage.

Zahlreiche Substrate und Produkte von Enzymreaktionen sind in Wasser oder Pufferlösungen nur begrenzt löslich. Aus wirtschaftlichen Gründen sollten aber für die technische Anwendung von Reaktionen mit trägergebundenen Enzymen möglichst konzentrierte Substratlösungen eingesetzt werden.

Es wurden daher zahlreiche Versuche mit Wasser/Lösungsmittelgemischen unternommen, um mit Enzymen höhere Produktkonzentrationen als in wässriger Lösung erzielen zu können.

Die Beobachtung zeigt, dass gelöste, solvatisierte Enzyme durch Lösungsmittel, wie Methanol, Ethanol, Aceton, Acetonitril, Dioxan und Dimethylformamid oder Dimethylsulfoxyd, teilweise oder vollständig unter partiellem oder vollständigem Verlust der Enzymaktivität denaturiert und aus der Lösung ausgefällt werden können.

Durch Bindung des Enzyms an einen polymeren Träger wird die Aggregation der Enzymmoleküle verhindert, so dass trägergebundene Enzyme in wesentlich geringerem Umfang als die freien Enzyme ihre Aktivität (K. Tamizawa und M.L. Bender, J. Biol. Chem. 249 (1974), 2130–2134) verlieren. Dennoch wird auch bei trägergebundenen Enzymen ein mehr oder minder starker Aktivitätsverlust des gebundenen Enzyms durch das Lösungsmittel beobachtet. Es wird entweder eine irreversible oder eine reversible Inaktivierung des Enzyms in Abhängigkeit von der Konzentration des Lösungsmittels beobachtet (H. Kaplan und K.J. Leidler, Canad. J. Chem. 45 (1967) 547–557, G.M. Umezurike, Biochem. J. 167 (1977), 831–833, T.N. Pattabiraman und W.B. Lawson, Biochem. J. 126 (1972), 645–657, G. Fink und H. Thoma, DECHEMA-Monographie 71, 295–314, H. Wan und C. Horvath, Biophys. Biochem. Acta 410 (1973), 135–140).

Die Enzymaktivität kann auch durch Veränderung der Porengrösse des Enzymträgers als Folge des Lösungsmitteleinflusses verändert werden.

Die oben erwähnten vollständig mit Wasser mischbaren Lösungsmittel weisen jedoch den weiteren technischen Nachteil auf, destillativ schwierig von Wasser abtrennbar zu sein.

Die Verwendung von mit Wasser nicht mischbaren Lösungsmitteln in Reaktionen mit trägergebundenen Enzymen ist bisher nur in einem Fall in der Literatur beschrieben (A.M. Klibanov, G.P. Samokhin, K. Martinek und I.V. Berezin, Biotechnol. Bioeng. 19 (1977) 1351–1361). In der zitierten Arbeit wird die Synthese von N-Acetyl-L-tryptophanethylester aus N-Acetyl-L-tryptophan und Ethanol mit kovalent an Glas gebundenem Chymotrypsin in Chloroform als Lösungsmittel beschrieben. Um die aus energetischen Gründen ungünstige Synthese des Esters durchführen zu können, muss in Abwesenheit von Wasser gearbeitet werden. Diese Literaturstelle gibt daher keinerlei Hinweis auf das erfindungsgemässe Verfahren.

Das erfindungsgemässe Verfahren vermeidet alle genannten Nachteile, schützt überdies die N-Acyl-aminosäureester vor unspezifischer Hydrolyse, so dass die enzymatische Spaltung auch bei höheren pH-Werten ohne Einbusse an Ausbeute durchgeführt werden kann.

Gegen die denaturierende Wirkung von Lösungsmitteln kann das trägergebundene Enzym nach kovalenter Bindung an einem polymeren Träger durch nachträgliche inter- und intramolekulare Quervernetzung mit bi- oder polyvalenten Reagenzien wie Glutardialdehyd oder anderen Reagenzien (F. Wold, Methods in Enzymology II, 617–640) geschützt werden.

Nach Beendigung der enzymatischen Reaktion wird nach dem Absitzen des Enzymharzes die organische Phase abgenommen und die wässrige Reaktionslösung portionsweise noch einmal mit dem zweifachen Volumen an organischem Lösungsmittel extrahiert. Die Extrakte werden vereinigt. Das Enzymharz wird abfiltriert und die verbleibende wässrige Phase beispielsweise mit Schwefelsäure sauer gestellt und mit Essigsäureäthylester extrahiert. Die erhaltenen Verbindungen werden dann auf ihre optische Reinheit überprüft.

Zur Herstellung der heterocyclischen D- bzw. L-Aminosäuren werden die Schutzgruppen $R_2$ und $R_4$ in der dem Fachmann geläufigen Weise abgespalten. Beispielsweise wird D-α-Furylglycin aus D-α-Formamidofurylessigsäuremethylester durch Erwärmen mit verdünnter Salzsäure gewonnen.

Beispiel 1

200 g Cellulose Avicel (Merck) werden in einer Lösung aus 500 ml Wasser und 500 ml Dioxan suspendiert und mit 20 g Cyanurchlorid versetzt. Der pH-Wert wird mit 2 N NaOH zwischen pH 7,0 und 9,0 gehalten. Nach 45 Minuten Rühren wird die aktivierte Cellulose über eine Fritte abgesaugt und in 800 ml Wasser suspendiert. Es werden 25 g Maxatase (Gist-Brocades N.V., Delft/Niederlande) hinzugefügt und es wird 20 Stunden bei Raumtemperatur und pH 7–8 gerührt. Danach wird der Enzymträger über eine Fritte abgesaugt, portionsweise mit destilliertem Wasser gewaschen und zuletzt trocken gesaugt.

Es werden 600 g feuchte Subtilisin-Cellulose erhalten. Aktivität: 284 ATEE (N-Acetyl-L-tyrosinethylester)-Einheiten/g Enzymträger. Gesamtaktivität: 170 250 ATEE-Einheiten, entsprechend 27,2% der eingesetzten Aktivität.

Beispiel 2

100 g DEAE-Cellulose (DE-52-Cellulose, Fa. Whatmann Ltd., Springfield/England) werden in ähnlicher Weise wie oben beschrieben mit Cyanurchlorid aktiviert. Davon werden 5 g nach Dialyse gegen Wasser lyophilisierte Maxatase oder Alcalase (659 Anson-Einheiten/g, Hersteller Novo AS, Kopenhagen/Dänemark) kovalent gebunden.

140 g feuchtes DE-52-Cellulose-Subtilisin mit 740 g ATEE-Einheiten/g Enzymträger werden nach Filtration erhalten.

Die gesamte Aktivitätsausbeute betrug 103 600 ATEE-Einheiten, entsprechend 16,5% der eingesetzten Aktivität.

Beispiel 3

20 g mit Aceton gewaschenes Anhydridharz (80 Gew.-% Tetraethylenglykoldimethacrylat, 10 Gew.-% Methacrylsäure und 10 Gew.-% Maleinsäureanhydrid) werden in 50 ml Wasser suspendiert. Es werden 40 ml 10 Gew.-%ige Hexamethylendiamin-Lösung bzw. Ethanolamin-Lösung bei pH 7,0 zugegeben und die Suspension über Nacht

bei pH 6,2 durch Titration konstant gehalten. Das Aminharz wird abgesaugt. Mit 1 M NaCl-Lösung wird überschüssiges Hexamethylendiamin ausgewaschen. Anschliessend wird mit entsalztem Wasser gewaschen.

Das Aminogruppen tragende Anhydridharz wird in 50 ml Wasser und 50 ml Dioxan suspendiert und 1 Stunde bei Raumtemperatur mit 2 g Cyanurchlorid bei pH 5,0 aktiviert. Das Harz wird mit Dioxan und Wasser gewaschen und 20 Stunden bei Raumtemperatur mit 2 g Maxatase bei pH 8,0 umgesetzt.

Es werden 48,2 g feuchtes Harz mit einer Aktivität von 126 ATEE-Einheiten/g Enzymträger erhalten.

Die gesamte Aktivitätsausbeute betrug 60 790 ATEE-Einheiten, entsprechend 12,1% der eingesetzten Aktivität.

Beispiel 4

300 g mit Diethylentriamin umgesetztes Polyacrylnitrilharz wird wie oben angegeben im gewichtsmässigen Verhältnis Träger: Cyanurchlorid (10 : 1) aktiviert und mit 30 g Maxatase bei 25 °C, pH 8,0 und 16 Stunden zur Umsetzung gebracht.

Es werden 288,5 g feuchtes Enzymharz erhalten.

Die proteolytische Aktivität am Träger betrug 81,0 ATEE-Einheiten/g Enzymharz, insgesamt 23 383 ATEE-Einheiten, entsprechend 3,1% der insgesamt eingesetzten Aktivität.

Die Beispiele 5 und 6 dienen lediglich zur Erklärung des beanspruchten Verfahrens. Auf die Erzeugnisse der Beispiele 5 und 6 sind keine Sachansprüche gerichtet.

Beispiel 5.1

D,L-α-Formamidofurylessigsäuremethylester

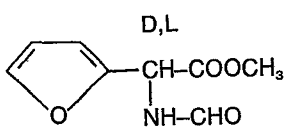

Zu 810 ml Essigsäureanhydrid werden bei 35 °C 390 ml Ameisensäure getropft. Anschliessend wird das Gemisch eine Stunde bei 55 °C gerührt. 14,1 g D,L-α-Furylglycin werden in 100 ml des obigen gemischten Anhydrids portionsweise eingetragen, die Temperatur soll dabei 30 °C nicht übersteigen. Ca. 6 Minuten nach der letzten Zugabe beginnt die Abscheidung der formylierten Aminosäure. Nach 3stündigem Rühren wird abgesaugt, zweimal mit Diäthyläther gewaschen und an der Luft getrocknet. Man erhält 13,2 g (78% d.Th.) beige Kristalle vom Schmelzpunkt 170–172 °C (Zers.).

13,0 g der vorstehend erhaltenen Verbindung werden in 40 ml Dimethylsulfoxid gelöst und unter Eiskühlung portionsweise mit einer Lösung von 3,08 g Natriumhydroxid in 17 ml Wasser versetzt. Dann werden 32,7 g Methyljodid zugegeben, wobei sich zwei Phasen bilden. Nach 50stündigem Rühren bei Raumtemperatur wird die Reaktionsmischung in 150 ml Wasser gegossen und fünfmal mit je 200 ml Methylenchlorid ausgeschüttelt. Die vereinigten organischen Phasen werden dann zweimal mit je 50 ml 2%iger Natriumsulfit-Lösung geschüttelt und über Natriumsulfat getrocknet. Nach dem Einengen erhält man 10,6 g (75,3% d.Th.) als Öl, das beim Animpfen kristallisiert; Schmp. 52–55 °C.

Beispiel 5.2

D-α-Formamidofurylessigsäuremethylester

30,0 g des nach Beispiel 5.1 erhaltenen Produktes werden in 800 ml destilliertem Wasser gelöst und bei pH 6,5 und 25 °C mit 132,5 g Cellulose-Subtilisin ($10^5$ ATEE-Einheiten) gespalten. Der pH-Wert wird mit 25%igem $NH_3$ konstant bei 6,5 gehalten. Nach 2,5 Stunden wird der Enzym-Träger abfiltriert und mit zweimal 100 ml Wasser gewaschen. Aus dem Filtrat wird die gewünschte Verbindung mit viermal 100 ml Methylenchlorid extrahiert. Die Ausbeute beträgt 11,2 g (74% d.Th.).

$$[\alpha]^{25\,°C}_{578nm} = -179{,}8°,\ c = 1 \text{ in Methanol}$$

Die wässrige Phase wird mit Schwefelsäure auf pH 1,5 eingestellt und viermal mit je 100 ml Essigsäureäthylester extrahiert. Die vereinigten Essigsäureäthylester-Extrakte werden im Vakuum zur Trockne eingedampft. Die Ausbeute an L-α-Formamidofurylessigsäure beträgt 11 g (79% d.Th.)

$$[\alpha]^{25\,°C}_{578\,nm} = -162{,}7°,\ c = 1 \text{ in Methanol}$$

Beispiel 5.3

D-α-Furylglycin-hydrochlorid

44,0 g des nach Beispiel 5.2 erhaltenen Produktes werden in der Wärme in 440 ml 2 N Salzsäure gelöst, 70 Minuten auf 80 °C gehalten, mit Tierkohle entfärbt und eingeengt.

Der Rückstand wird mit einem Gemisch aus 80 Teilen Aceton und 40 Teilen Äthanol digeriert und abfiltriert. Das Produkt wird mit Aceton gewaschen und im Vakuum getrocknet. Man erhält 26,0 g (61% d.Th.) D-α-Furylglycinhydrochlorid mit einem Schmelzpunkt 193–195 °C (Zers.).

$$[\alpha]^{25\,°C}_{578\,nm} = -114{,}9°,\ c = 1 \text{ in Methanol}$$

In entsprechender Weise wir L-α-Furylglycin-hydrochlorid gewonnen.

Beispiel 6.1

D,L-α-t-Butoxycarbonylaminofurylessigsäure

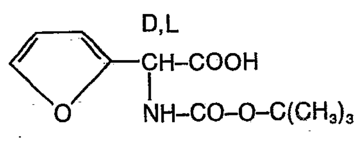

5,0 g DL-α-Furylglycin werden in 100 ml 80%igem wässrigem Dioxan mit 4 N Natronlauge auf pH 8 gebracht. Man gibt 8,0 g 2-(t-Butoxycarbonyl-oximino)-2-phenylacetonitril zu und erwärmt 2 Stunden auf 70 °C. Während dieser Zeit wird durch Zugabe von 4 N Natronlauge der obige pH aufrechterhalten. Danach werden 80 ml Wasser zu-

gegeben, das Dioxan abdestilliert und mehrere Male mit Essigsäureäthylester extrahiert. Die wässrige Phase wird mit 10%iger Zitronensäure auf pH 4 gebracht, mit Kochsalz gesättigt und mit Essigester ausgeschüttelt. Die Essigesterphasen werden über Natriumsulfat getrocknet und eingeengt. Der Rückstand wird aus wenig absolutem Tetrachlorkohlenstoff umkristallisiert. Man erhält 4,3 g D,L-α-t-Butoxycarbonylaminofurylessigsäure vom Schmp. 99–101 °C.

Beispiel 6.2
D.L-α-t-Butoxycarbonylaminofurylessigsäuremethylester

Zu einer Lösung von 6,0 g der nach Beispiel 6.1 erhaltenen Verbindung in 100 ml absolutem Diäthyläther wird bis zur bleibenden Gelb-Färbung ätherische Diazomethan-Lösung getropft. Dann wird mit verdünnter Natronlauge ausgeschüttelt, mit Wasser gewaschen, über Natriumsulfat getrocknet und eingedampft. Man erhält 6,1 g (96% d.Th.) der gewünschten Verbindung vom Schmp. 63–68 °C.

Beispiel 6.3
Enzymatische Spaltung von D,L-α-t-Butoxycarbonylaminofurylessigsäuremethylester in Gegenwart von Methylisobutylketon

20 g der nach Beispiel 6.2 erhaltenen Verbindung werden in einem Gemisch aus 800 ml Wasser und 200 ml Methylisobutylketon gelöst und mit dem nach Beispiel 4 hergestellten Subtilisin-Trägerharz bei pH 7,0 und 37 °C unter Rühren gespalten. Der pH-Wert wird mit 25%igem Ammoniak konstant gehalten. Nach 5 Stunden Reaktionszeit wird der Rührer abgestellt und die organische Phase von der wässrigen Phase abgetrennt. Anschliessend wird die wässrige Phase noch zweimal mit je 200 ml Methylisobutylketon extrahiert. Die vereinigten organischen Phasen werden im Vakuum zur Trockne eingedampft. Die Ausbeute an D-α-t-Butoxycarbonylaminofurylessigsäuremethylester beträgt 10,2 g (68% d.Th.).

$[\alpha]^{25\,°C}_{578\,nm} = -81{,}4°$, c = 1 in Methanol

Die verbleibende wässrige Phase wird mit Schwefelsäure auf pH 1,5 eingestellt und mit viermal 100 ml Essigsäureäthylester extrahiert.

Die Ausbeute an L-α-t-Butoxycarbonylaminofurylessigsäure beträgt 8,8 g (93% d.Th.).

$[\alpha]^{25\,°C}_{578\,nm} = +104{,}7°$, c = 1 in Methanol

Beispiel 6.4
D-α-Furylglycin-hydrochlorid

10,0 g D-α-t-Butoxycarbonylaminofurylessigsäuremethylester werden in einem Gemisch aus 100 ml 2 N Salzsäure und 45 ml Dioxan warm gelöst, 2 Stunden auf 80 °C gehalten, mit Aktivkohle behandelt und eingedampft. Der Rückstand wird mit einem Gemisch aus 20 ml Aceton und 10 ml Äthanol digeriert, abfiltriert, mit Aceton gewaschen und getrocknet. Man erhält 3,9 g D-α-Furylglycin-hydrochlorid.

$[\alpha]^{25\,°C}_{578\,nm} = -109{,}2°$, c = 1 in Methanol

Beispiel 7.1
D,L-α-Formamido-(5-methyl-isoxazol-3-yl)-essigsäuremethylester

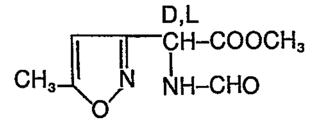

wurde analog Beispiel 5.1 hergestellt.

Beispiel 7.2
D-α-Formamido-65-methyl-isoxazol-3-yl)-essigsäuremethylester wurde analog Beispiel 5.2 hergestellt.

Beispiel 7.3
D-α-(5-Methyl-isoxazol-3-yl)-glycin-hydrochlorid

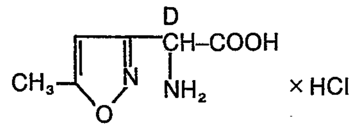

wurde analog Beispiel 5.3 hergestellt.

Beispiel 8.1
D,L-α-Formamido-pyrid-3-yl-essigsäuremethylester

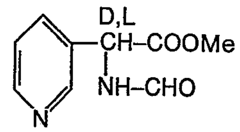

wurde analog Beispiel 5.1 hergestellt.

Beispiel 8.2
D-α-Formamido-pyrid-3-yl-essigsäuremethylester wurde analog Beispiel 5.2 hergestellt.

Beispiel 8.3
D-α-Pyrid-3-yl-glycin-dihydrochlorid

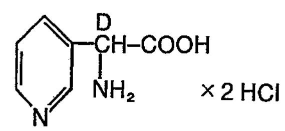

wurde analog Beispiel 5.3 hergestellt.

Beispiel 9.1
D,L-α-Formamido-thiazol-2-yl-essigsäuremethylester

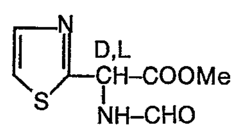

wurde analog Beispiel 5.1 hergestellt.

Beispiel 9.2
D-α-Formamido-thiazol-2-yl-essigsäuremethylester wurde analog Beispiel 5.2 hergestellt.

Beispiel 9.3

D-α-Thiazol-2-yl-glycin-hydrochlorid

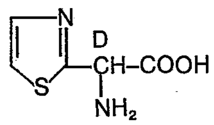

× HCl

wurde analog Beispiel 5.3 hergestellt.

Beispiel 10.1

D,L-α-Formamido-(5-chlorfuryl)-essigsäuremethylester

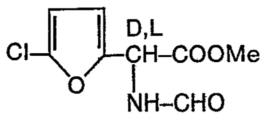

wurde analog Beispiel 5.1 hergestellt.

Beispiel 10.2

D-α-Formamido-(5-chlorfuryl)-essigsäuremethylester wurde analog Beispiel 5.2 hergestellt.

Beispiel 10.3

D-α-s-Chlorfuryl-glycin-hydrochlorid

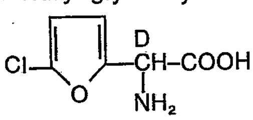

× HCl

wurde analog Beispiel 5.3 hergestellt.

**Patentansprüche**

1. Verfahren zur Herstellung von Verbindungen der Formel I

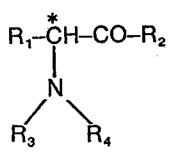

(I)

worin $R_1$ einen gesättigten oder ungesättigten, gegebenenfalls substituierten heterocyclischen Rest, der 1 bis 4 gleiche oder verschiedene Heteroatome aus der Reihe Sauerstoff, Schwefel und/oder Stickstoff enthält und gegebenenfalls benzokondensiert ist,

$R_2$ Hydroxy, $C_1$–$C_4$-Alkoxy oder $N(R_5)_2$,

$R_3$ und $R_4$ Wasserstoff, Acyl oder durch $C_1$–$C_4$-Alkoxycarbonyl substituiertes $C_2$–$C_4$-Alkenyl und

$R_5$ Wasserstoff oder $C_1$–$C_4$-Alkyl bedeuten und das mit* gekennzeichnete C-Atom entweder in der D- oder L-Form vorliegt, dadurch gekennzeichnet, dass man auf die racemischen Verbindungen der Formel I trägergebundene proteolytische Enzyme aus der Gruppe Subtilisin, α-Chymotrypsin, Papain, Ficin, Bromelain oder aus Bacillus subtilis oder Bacillus licheniformis isolierte proteolytische Enzyme in einem zweiphasigen Medium, bestehend aus Wasser und einem mit Wasser nicht mischbaren Lösungsmittel, einwirken lässt, die unveränderten D-Verbindungen der Formel I, worin $R_2$ nicht Hydroxy und $R_4$ nicht Wasserstoff ist, von den L-Verbindungen der Formel I, worin $R_2$ Hydroxy ist und $R_4$ nicht Wasserstoff ist, in üblicher Weise abtrennt und die voneinander getrennten D- und L-Verbindungen gegebenenfalls in üblicher Weise in Verbindungen der Formel I, worin $R_2$ Hydroxy und $R_3$ und $R_4$ Wasserstoff bedeuten, überführt.

2. Verfahren nach Anspruch 1, dadurch gekennzeichnet, dass man als wasser-nicht-mischbares Lösungsmittel Methylenchlorid, Chloroform, Toluol, Benzol, Essigsäureethylester, Petrolether, Methylisobutylketon oder Isobutanol einsetzt.

3. Verfahren nach Anspruch 1 oder 2, dadurch gekennzeichnet, dass man als Träger des Enzyms Cellulosen, derivatisierte Cellulosen, amino- oder hydroxylgruppenhaltige Polyacrylamidgele oder durch Amino- oder Hydroxylgruppen modifizierte Anhydridharze einsetzt.

4. D-α-(5-Methyl-isoxazol-3-yl)-glycin-hydrochlorid.

5. D-α-Pyrid-3-yl-glycin-dihydrochlorid.

6. D-α-Thiazol-2-yl-glycin-hydrochlorid.

7. D-α-5-Chlorfuryl-glycin-hydrochlorid.

8. Verwendung der nach den Ansprüchen 1–3 hergestellten Verbindungen zur Herstellung optisch reiner acylierter β-Lactamantibiotika.

**Claims**

1. Process for the preparation of compounds of the formula I

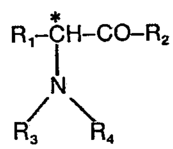

(I)

wherein

$R_1$ denotes a saturated or unsaturated optionally substituted heterocyclic radical which contains 1 to 4 identical or different hetero atoms from the series comprising oxygen, sulphur and/or nitrogen and is optionally benzo-fused,

$R_2$ denotes hydroxyl, $C_1$–$C_4$-alkoxy or $N(R_5)_2$,

$R_3$ and $R_4$ denote hydrogen, acyl or $C_2$–$C_4$-alkenyl which is substituted by $C_1$–$C_4$-alkoxycarbonyl and

$R_5$ denotes hydrogen or $C_1$–$C_4$-alkyl and the C atom marked with * is either in the D-form or the L-form, characterised in that carrier-bonded proteolytic enzymes from the group comprising subtilisin, α-chymotrypsin, papain, ficin, bromelain or proteolytic enzymes isolated from Bacillus subtilis or Bacillus licheniformis are allowed to act on the racemic compounds of the formula I in a two-phase medium consisting of water and a water-immiscible solvent, the unchanged D-compounds of the formula I, wherein $R_2$ ist not hydroxyl and $R_4$ is not hydrogen, are separated from the L-compounds of the formula I, wherein $R_2$ is hydroxyl and $R_4$ is not hydrogen, in the customary manner, and the D- and L-compounds which have been separated from one another are optionally converted into compounds of the formula I, wherein $R_2$ denotes hydroxyl and $R_3$ and $R_4$ denote hydrogen, in the customary manner.

2. Process according to Claim 1, characterised in that the water-immiscible solvent used is methylene chloride, chloroform, toluene, ben-

zene, ethyl acetate, petroleum ether, methyl isobutyl ketone or isobutanol.

3. Process according to Claim 1 or 2, characterised in that the carriers employed for the enzyme are celluloses, cellulose derivatives, polyacrylamide gels containing amino groups or hydroxyl groups or anhydride resins modified by amino groups or hydroxyl groups.

4. D-α-(5-Methyl-isoxazol-3-yl)-glycine hydrochloride.

5. D-α-Pyrid-3-yl-glycine dihydrochloride.

6. D-α-Thiazol-2-yl-glycine hydrochloride.

7. D-α-5-Chlorofuryl-glycine hydrochloride.

8. Use of the compounds prepared according to Claims 1–3 for the preparation of optically pure acylated β-lactam antibiotics.

**Revendications**

1. Procédé de préparation de composés de formule I:

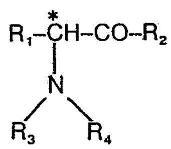

(I)

dans laquelle $R_1$ représente un radical hétérocyclique saturé ou insaturé éventuellement substitué qui contient 1 à 4 hétéroatomes identiques ou différents de la série de l'oxygène, soufre et/ou azote, et qui est éventuellement benzocondensé,

$R_2$ hydroxy, alcoxy en $C_1$–$C_4$ ou N $(R_5)_2$,

$R_3$ et $R_4$ de l'hydrogène, acyle ou alcényle en $C_2$–$C_4$ substitué par un alcoxy ($C_1$–$C_4$)carbonyle et

$R_5$ de l'hydrogène ou un alcoyle en $C_1$–$C_4$, tandis que l'atome de carbone marqué par * se présente dans la forme D ou la forme L, caractérisé en ce qu'on fait agir sur les composés racémiques de formule I des enzymes protéolytiques liées à un support, du groupe de la subtilisine, α-chymotrypsine, papaïne, ficine, bromélaïne ou les enzymes protéolytiques isolées à partir du Bacillus subtilis ou du Bacillus licheniformis dans un milieu à deux phases consistant en de l'eau et en un solvant non miscible avec l'eau, en ce qu'on sépare de la manière usuelle les composés D non modifiés de formule I, dans laquelle $R_2$ n'est pas un hydroxy et $R_4$ n'est pas de l'hydrogène, d'avec le composé L de formule I dans laquelle $R_2$ est un hydroxy et $R_4$ n'est pas de l'hydrogène et en ce que l'on convertit éventuellement les composés D et L mutuellement séparés de la manière usuelle en composés de formule I dans laquelle $R_2$ signifie un hydroxy et $R_3$ et $R_4$ de l'hydrogène.

2. Procédé selon la revendication 1, caractérisé en ce qu'on utilise en tant que solvants non miscibles avec l'eau du chlorure de méthylène, chloroforme, toluène, benzène, acétate d'éthyle, éther de pétrole, méthylisobutylcétone ou isobutanol.

3. Procédé selon la revendication 1 ou 2, caractérisé en ce qu'on utilise comme support de l'enzyme des celluloses, des dérivés de cellulose, des gels de polyacrylamide portant des groupes amino ou hydroxyle ou des résines d'anhydride modifiées par des groupes amino ou hydroxyle.

4. Chlorhydrate de D-α-(5-méthyl-isoxazol-3-yl)-glycine.

5. Dichlorhydrate de D-α-pyrid-3-yl-glycine.

6. Chlorhydrate de D-α-thiazol-2-yl-glycine.

7. Chlorhydrate de D-α-5-chlorofurylglycine.

8. Utilisation des composés préparés selon les revendications 1 à 3 pour la préparation d'antibiotiques de β-lactame acylés optiquement purs.